# EUROPEAN PATENT APPLICATION

(11) **EP 4 640 683 A1**
(43) Date of publication of application: **29.10.2025**
(21) Application number: 23906103.9
(22) Date of filing: 22.12.2023
(51) Int. Cl.: C07D 417/12

(54) **METHOD FOR PREPARING P2X3 INHIBITOR**

(30) Priority: 22.12.2022 CN 202211667185
(71) Applicant: Humanwell Healthcare (Group) Co., Ltd., Wuhan, Hubei 430075 (CN); Wuhan Humanwell Innovative Drug Research and Development Center Limited Company, Wuhan, Hubei 430075 (CN)
(72) Inventor: ZHANG, Xuejun, Wuhan, Hubei 430075 (CN); ZANG, Yang, Wuhan, Hubei 430075 (CN); LI, Qun, Wuhan, Hubei 430075 (CN); ZHANG, Bo, Wuhan, Hubei 430075 (CN); LI, Yuan, Wuhan, Hubei 430075 (CN); YANG, Qiongfeng, Wuhan, Hubei 430075 (CN); XIA, Qingfeng, Wuhan, Hubei 430075 (CN); YUE, Yang, Wuhan, Hubei 430075 (CN); YANG, Jun, Wuhan, Hubei 430075 (CN); LI, Li'e, Wuhan, Hubei 430075 (CN)
(74) Representative: Gille Hrabal Partnerschaftsgesellschaft mbB Patentanwälte
(86) International application number: PCT/CN2023/141078
(87) International publication number: WO 2024/131948

(57) **Abstract**

The present invention belongs to the field of drug synthesis. Specifically, disclosed is a method for preparing a P2X3 inhibitor, i.e., 2-fluoro-5-(((2S,3R)-3-hydroxybutan-2-yl)oxy)-3(5-methylthiazol-2-yl)-N-((R)-1-(2-(trifluoromethyl)pyrimidin-5-yl)ethyl)benzamide (I-1). The method comprises the following steps: using 3-bromo-2-fluoro-5-iodobenzoic acid as a starting material, substituting iodine with a hydroxyl group and subjecting a carboxyl group to methyl esterification to obtain a compound 1C; subjecting the compound 1C and B₂(pin)₂ to a coupling reaction to obtain 1D; subjecting the compound 1D and 2-bromo-5-methylthiazole to a coupling reaction to obtain 1E; substituting the compound 1E with 1F to then obtain a compound 1H; hydrolyzing 1H under the action of a base to obtain 1I, and subjecting the compound 1I and 1L to a condensation reaction to obtain a product 1-1. The synthesis route provided by the present invention makes it easy to control product quality, has a high yield and is suitable for industrial production.

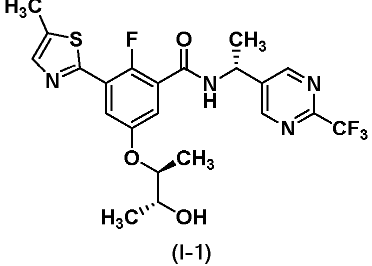

## Description

The present application claims priority to Chinese Patent Application No. 2022116671852 filed with China National Intellectual Property Administration on Dec. 22, 2022 and entitled "METHOD FOR PREPARING P2X3 INHIBITOR", which is incorporated herein by reference in its entirety.

### TECHNICAL FIELD

The present disclosure relates to a process for synthesizing 2-fluoro-5-(((2S,3R)-3-hydroxybutan-2-yl)oxy)-3-(5-methylthiazol-2-yl)-N-((R)-1-(2-(trifluoromethyl)pyrimidin-5-yl)ethyl)benzamide and intermediates thereof.

### BACKGROUND

P2X receptor is a non-selective ATP-gated ion channel receptor, i.e., a purinergic receptor, which can bind to extracellular ATPs mainly derived from damaged or inflamed tissues. This receptor is widely expressed in the nervous, immune, cardiovascular, skeletal, gastrointestinal, respiratory, and endocrine systems and other systems, and is involved in a variety of physiological processes such as the regulation of heart rhythm and contractility, the regulation of vascular tone, the regulation of nociception (especially chronic pain), the contraction of vas deferens during ejaculation, the contraction of bladder during urination, the aggregation of platelets, the activation of macrophages, apoptosis, neuron-glial interactions, and the like. The P2X receptor described above includes: seven homologous receptors, i.e., P2X1, P2X2, P2X3, P2X4, P2X5, P2X6, and P2X7; and three heterologous receptors, i.e., P2X2/3, P2X4/6, and P2X1/5.

P2X3 is a subtype of the P2X receptor family, and is selectively expressed in dorsal root ganglia, spinal cord, and brain neurons of nerve endings, i.e., in primary sensory neurons with small to medium diameters.

Numerous studies have shown that activation of P2X3 and P2X2/3 expressed in the primary sensory neurons plays an important role in acute injury, hyperalgesia, and hypersensitivity in rodents. Many studies have shown that upregulation of P2X3 receptor expression may lead to the development of hyperalgesia and is involved in pain signaling. P2X3-knockout mice exhibit reduced pain responses, and in models of pain and inflammatory pain, P2X3 receptor antagonists demonstrate an alleviating effect on nociception.

P2X3 is distributed in primary afferent nerves around the airways and is capable of regulating cough. Studies have shown that ATPs released from damaged or inflamed airway tissues act on P2X3 receptors of primary neurons, triggering depolarization and action potentials. The propagation of these potentials causes the impulse to cough and thus induces coughing. P2X3 receptors play an important role in cough reflex hypersensitivity. By antagonizing the binding of ATP to P2X3 receptors, the hypersensitivity of the cough reflex can be inhibited, thereby suppressing excessive coughing in patients with chronic cough. In addition, studies have also shown that P2X3 antagonists can treat chronic obstructive pulmonary disease, pulmonary fibrosis, pulmonary arterial hypertension, or asthma. Therefore, P2X3 antagonists are also promising to become new drugs for the treatment of the diseases described above.

It has been reported that P2X3 is involved in the afferent pathway that controls the bladder capacity reflex, and P2X3-knockout mice exhibit a significant decrease in urination frequency and a significant increase in bladder capacity. Therefore, the binding of P2X3 receptor-inhibiting antagonists to P2X3 receptors has effects in the treatment of urinary storage disorders and voiding disorders, such as overactive bladder. Therefore, P2X3 antagonists may be potential drugs for the treatment of overactive bladder and other related diseases.

P2X3 antagonists show great promise. Currently, commonly used clinical cough drugs gabapentin, morphine, and amitriptyline or treatment with speech pathology can alleviate cough in many patients, but these therapies are not suitable for all patients. In addition, centrally acting drugs such as gabapentin and the like may cause adverse side effects and thus are not suitable for long-term medication. There is an urgent clinical need to develop chronic refractory cough drugs suitable for long-term medication, so as to provide doctors with more medication options. Therefore, the development of P2X3 antagonists is of great clinical significance.

### SUMMARY

The present disclosure provides a method for preparing 2-fluoro-5-(((2S,3R)-3-hydroxybutan-2-yl)oxy)-3-(5-methylthiazol-2-yl)-N-((R)-1-(2-(trifluoromethyl)pyrimidin-5-yl)ethyl)benzamide and an intermediate thereof. The synthetic route provided by the present disclosure facilitates quality control, achieves high yield, and is suitable for industrial production.

The present disclosure provides a method for preparing compound I-1, which comprises the following step:
a2) subjecting compound 1I and compound 1L to a condensation reaction as shown below under the action of a condensing agent and a base in a solvent to give compound I-1;

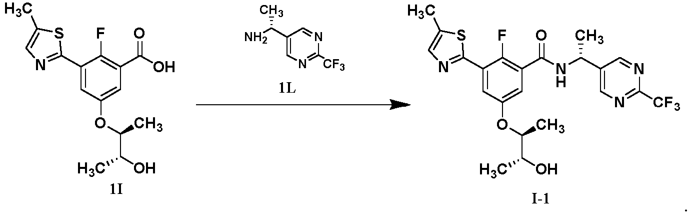

In an optional embodiment of the present disclosure, in step a2, the solvent includes, but is not limited to, water, a chlorinated alkane solvent, an ether solvent, an amide solvent, an arene solvent, an ester solvent, or a mixture of any two or more thereof; preferably, the solvent is an amide solvent. The chlorinated alkane solvent is preferably dichloromethane, chloroform, dichloroethane, or a mixture of any two or more thereof, further preferably dichloromethane. The ether solvent is preferably tetrahydrofuran, diethyl ether, 1,4-dioxane, anisole, methyl tert-butyl ether, or a mixture of any two or more thereof, further preferably tetrahydrofuran. The amide solvent is preferably N,N-dimethylformamide, N,N-dimethylacetamide, or a mixture thereof, further preferably N,N-dimethylformamide. The arene solvent is preferably toluene. The ester solvent is preferably ethyl acetate. In an optional embodiment of the present disclosure, in the method for preparing the compound of formula I-1, the solvent is preferably an amide solvent, more preferably N,N-dimethylformamide.

In an optional embodiment of the present disclosure, in step a2, compound 1L is present as a free base or a salt thereof, such as a hydrochloride salt of compound 1L.

In an optional embodiment of the present disclosure, in step a2, the condensing agent may be one or more of HOBt/EDCI, T₃P, PyBOP, DCC, CDI, or HATU; preferably, the condensing agent is HOBt/EDCI.

In an optional embodiment of the present disclosure, in step a2, the base may be DIPEA, sodium bicarbonate, potassium carbonate, cesium carbonate, triethylamine, or pyridine; preferably, the base is DIPEA.

In an optional embodiment of the present disclosure, in step a2, the condensing agent is HOBt/EDCI, the base is DIPEA, and the solvent is DMF.

In an optional embodiment of the present disclosure, in step a2, a molar ratio of intermediate 1I to the condensing agent may be 1:(1-5), preferably 1:(1.5-2), and more preferably 1:1.5.

In an optional embodiment of the present disclosure, in step a2, a molar ratio of intermediate 1I to the base may be 1:(1-5), preferably 1:(2-4), and more preferably 1:3.

In an optional embodiment of the present disclosure, in step a2, a molar ratio of intermediate 1I to intermediate 1L may be 1:(1-4), preferably 1:(1-3), and more preferably 1:1.2.

In an optional embodiment of the present disclosure, in step a2, the condensation reaction is carried out under an atmosphere of an inert gas, and the inert gas may be nitrogen, helium, or argon.

In an optional embodiment of the present disclosure, a reaction time for the condensation reaction may be 1 h to 20 h, such as 1 h to 2 h.

In an optional embodiment of the present disclosure, in step a2, after the completion of the condensation reaction, a post-treatment step may be further comprised, for example: subjecting a reaction liquid to extraction, centrifugation, drying, recrystallization, centrifugation, and drying.

In an optional embodiment of the present disclosure, in step a2, the extraction in the post-treatment step comprises adding ethyl acetate to the reaction liquid, adding a 6% aqueous sodium bicarbonate solution for washing twice, collecting the organic phase, washing once with a 0.2 M aqueous hydrochloric acid solution until pH = 3 to 4, collecting the organic phase, washing twice with purified water until the pH is neutral, and collecting the organic phase.

In an optional embodiment of the present disclosure, in step a2, the drying in the post-treatment step comprises drying the centrifuged filter cake in a blast air oven at 55±5 °C for not less than 8 h.

In an optional embodiment of the present disclosure, in step a2, the recrystallization comprises dissolving the dried product in a good solvent under heating, dropwise adding an anti-solvent, cooling, and crystallizing.

In an optional embodiment of the present disclosure, in step a2, the good solvent may be one or more of methanol, absolute ethanol, isopropanol, ethyl acetate, and isopropyl acetate; preferably, the good solvent is absolute ethanol. In an optional embodiment of the present disclosure, in step a2, the anti-solvent may be one or two or more of isopropyl ether, n-heptane, n-hexane, and petroleum ether; preferably, the anti-solvent is n-heptane.

In an optional embodiment of the present disclosure, in step a2, a temperature for heating and dissolving during the recrystallization is 60 °C to 80 °C.

In an optional embodiment of the present disclosure, in step a2, the temperature is decreased to 15 °C to 25 °C during the recrystallization.

The method for preparing compound I-1 may further comprise a method for preparing compound 1I, which comprises the following step:
a1) subjecting compound 1H to a hydrolysis reaction as shown below in the presence of a basic substance in a solvent to give compound 1I;

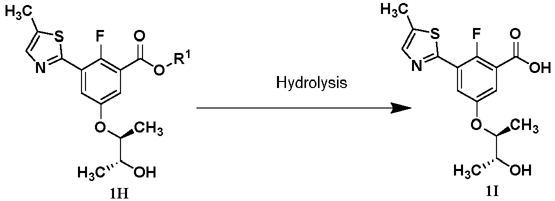

In an optional embodiment of the present disclosure, R¹ is C₁-C₄ alkyl or benzyl, such as methyl or ethyl.

In an optional embodiment of the present disclosure, in step a1, the solvent is selected from an alcohol solvent, water, and a mixed solvent of an alcohol solvent and water. The alcohol solvent is preferably methanol or ethanol.

In an optional embodiment of the present disclosure, in the method for preparing the compound of formula 1I, the solvent is preferably a mixed solvent of an alcohol solvent and water, more preferably a mixed solvent of methanol and water.

In an optional embodiment of the present disclosure, in step a1, the basic substance may be one or more of triethylamine, potassium tert-butoxide, sodium tert-butoxide, lithium carbonate, potassium carbonate, sodium carbonate, sodium hydroxide, lithium hydroxide, or potassium hydroxide; preferably, the basic substance is lithium hydroxide.

In an optional embodiment of the present disclosure, in step a1, R¹ is methyl, the basic substance is lithium hydroxide, and the solvent is a mixed solvent of methanol and water.

In an optional embodiment of the present disclosure, in step a1, a mixing volume ratio of methanol to water is (1-10):1.

In an optional embodiment of the present disclosure, in step a1, a molar ratio of compound 1H to the basic substance is 1:(1-5), preferably 1:2.

In an optional embodiment of the present disclosure, in step a1, a reaction time for the hydrolysis reaction may be 1 h to 20 h, such as 1 h to 2 h.

In an optional embodiment of the present disclosure, in step a1, after the completion of the hydrolysis reaction, a post-treatment step may be further comprised, for example: subjecting a reaction liquid to cooling, acidification, centrifugation, washing, and drying to give pure compound 1I.

In an optional embodiment of the present disclosure, in step a1, the post-treatment step comprises cooling the reaction liquid to 0 °C to 20 °C.

In an optional embodiment of the present disclosure, in step a1, the acidification in the post-treatment step comprises acidifying the reaction liquid until pH is 4, crystallizing at a maintained temperature of 0-20 °C, further acidifying until the pH is 2 to 3, and crystallizing at a maintained temperature.

The method for preparing compound 1I may further comprise a method for preparing compound 1H, which comprises the following step:
b3) subjecting compound 1E to a substitution reaction as shown below with 1F in the presence of a base in a solvent, followed by acidification with an acid, to give compound 1H:

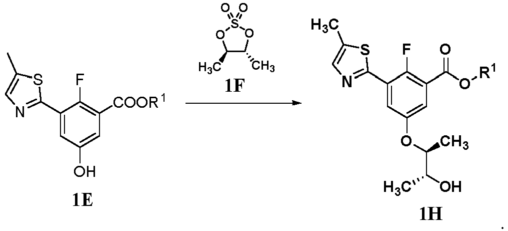

In an optional embodiment of the present disclosure, R¹ is C₁-C₄ alkyl or benzyl, such as methyl or ethyl.

In an optional embodiment of the present disclosure, in step b3, the solvent is selected from an alcohol solvent, an aromatic hydrocarbon solvent, an ether solvent, an amide solvent, and a mixture of any two or more thereof; preferably, the solvent is a mixture of an ether solvent and an amide solvent. The alcohol solvent is preferably methanol, ethanol, or a mixture thereof. The aromatic hydrocarbon solvent is preferably toluene. The ether solvent is preferably 1,4-dioxane, tetrahydrofuran, or a mixture thereof, further preferably tetrahydrofuran. The amide solvent is preferably N,N-dimethylformamide, N,N-dimethylacetamide, or a mixture thereof.

In an optional embodiment of the present disclosure, in the method for preparing the compound of formula 1H, the solvent is preferably a mixture of an ether solvent and an amide solvent, more preferably a mixture of tetrahydrofuran and N,N-dimethylformamide.

In an optional embodiment of the present disclosure, in step b3, the base may be one or more of DIPEA, triethylamine, pyridine, cesium carbonate, potassium carbonate, potassium phosphate, potassium acetate, sodium hydride, sodium hydroxide, and potassium hydroxide; preferably, the base is potassium carbonate.

In an optional embodiment of the present disclosure, in step b3, the acid may be sulfuric acid, preferably a 20%-75% aqueous sulfuric acid solution, and more preferably a 20% aqueous sulfuric acid solution.

In an optional embodiment of the present disclosure, in step b3, R¹ is methyl, the solvent is a mixture of tetrahydrofuran and N,N-dimethylformamide, the base is potassium carbonate, and the acid is a 20% aqueous sulfuric acid solution.

In an optional embodiment of the present disclosure, in step b3, a molar ratio of compound 1E to compound 1F is 1:(1-5), preferably 1:(1.3-1.5), and more preferably 1:1.3.

In an optional embodiment of the present disclosure, in step b3, a molar ratio of compound 1E to the base is 1:(1-5), preferably 1:2.

In an optional embodiment of the present disclosure, in step b3, a mixing volume ratio of tetrahydrofuran to N,N-dimethylformamide is (1-10):1.

In an optional embodiment of the present disclosure, in step b3, a molar ratio of compound 1E to the acid is 1:(1-5), preferably 1:2.

In an optional embodiment of the present disclosure, in step b3, a reaction temperature for the substitution reaction may be 60 °C to 75 °C, preferably 65 °C to 70 °C.

In an optional embodiment of the present disclosure, in step b3, a temperature for the acidification is 50 °C to 70 °C, preferably 55 °C to 65 °C.

In an optional embodiment of the present disclosure, in step b3, a reaction time for the substitution reaction may be 5 h to 20 h, such as 12 h.

In an optional embodiment of the present disclosure, in step b3, after the completion of the substitution reaction, a post-treatment step may be further comprised, for example: subjecting a reaction liquid to cooling, phase separation by standing, extraction, washing, concentration, recrystallization, centrifugation, and washing to give wet compound 1H.

In an optional embodiment of the present disclosure, in step b3, the recrystallization comprises dissolving the dried product in a good solvent under heating, dropwise adding an anti-solvent, cooling, and crystallizing.

In an optional embodiment of the present disclosure, in step b3, the good solvent may be one or more of methanol, absolute ethanol, isopropanol, ethyl acetate, and isopropyl acetate; preferably, the good solvent is methanol.

In an optional embodiment of the present disclosure, in step b3, the anti-solvent may be one or two or more of water, isopropyl ether, n-heptane, n-hexane, and petroleum ether; preferably, the anti-solvent is water.

In an optional embodiment of the present disclosure, in step b3, a temperature for heating and dissolving during the recrystallization is 50 °C to 70 °C.

In an optional embodiment of the present disclosure, in step b3, the temperature is decreased to 10 °C to 20 °C during the recrystallization.

In an optional embodiment of the present disclosure, step b3 comprises the following steps: adding compound 1E, compound 1F, and a base to a solvent for a first-stage reaction, and then adding an acid to the reaction system for acidification to give compound 1H.

The method for preparing compound 1H may further comprise a method for preparing compound 1E, which comprises the following step:
b2) subjecting compound 1D to a coupling reaction as shown below with 2-bromo-5-methylthiazole in the presence of a palladium catalyst and a base in a solvent to give compound 1E:

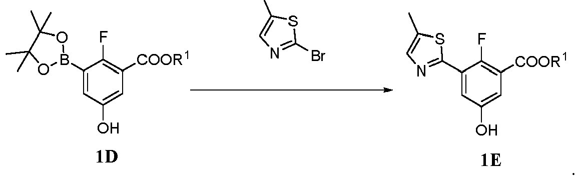

In an optional embodiment of the present disclosure, R¹ is C₁-C₄ alkyl or benzyl, such as methyl or ethyl.

In an optional embodiment of the present disclosure, in step b2, the solvent is selected from water, an alcohol solvent, an aromatic hydrocarbon solvent, an ether solvent, and a mixture of any two or more thereof; preferably, the solvent is an ether solvent. The alcohol solvent is preferably methanol, ethanol, or a mixture thereof; the aromatic hydrocarbon solvent is preferably toluene; the ether solvent is preferably ethylene glycol dimethyl ether, tetrahydrofuran, or a mixture thereof. In an optional embodiment of the present disclosure, in the method for preparing the compound of formula 1E, the solvent is preferably an ether solvent, more preferably ethylene glycol dimethyl ether.

In an optional embodiment of the present disclosure, in step b2, the palladium catalyst may be one or more of Pd(OAc)₂, PdCb, Pd(TFA)₂, Pd[O₂C(CH₃)₃]₂, Pd₂(dba)₃, PdBr₂, and Pd(dppf)Cl₂; preferably, the palladium catalyst is Pd(OAc)₂.

In an optional embodiment of the present disclosure, in step b2, the base may be one or more of KHCO₃, NaHCO₃, Na₂CO₃, Ba(OH)₂, K₃PO₄, Cs₂CO₃, K₂CO₃, NaOH, KOH, Et₃N, DIPEA, NaOMe, NaOEt, t-BuOK, t-BuONa, NaH, LiHMDS, NaHMDS, potassium acetate, sodium tert-amylate, n-butyllithium, diethylamine, and dicyclohexylamine; preferably, the base is K₂CO₃.

In an optional embodiment of the present disclosure, in step b2, the coupling reaction further comprises a phosphine ligand, and the phosphine ligand may be one or more of PPh₃, SPhos, XPhos, 1,1'-bis(diphenylphosphino)ferrocene, and XantPhos; preferably, the phosphine ligand is XantPhos.

In an optional embodiment of the present disclosure, in step b2, R¹ is methyl, the solvent is ethylene glycol dimethyl ether, the palladium catalyst is Pd(OAc)₂, the base is K₂CO₃, and the phosphine ligand XantPhos is comprised.

In an optional embodiment of the present disclosure, in step b2, a molar ratio of intermediate 1D to the palladium catalyst is 1:(0.01-0.1), preferably 1:(0.01-0.05), and more preferably 1:0.02.

In an optional embodiment of the present disclosure, in step b2, a molar ratio of intermediate 1D to 2-bromo-5-methylthiazole is 1:(0.5-5), preferably 1:1.

In an optional embodiment of the present disclosure, in step b2, a molar ratio of intermediate 1D to the base is 1:(1-5), preferably 1:(1.5-2), and more preferably 1:1.9.

In an optional embodiment of the present disclosure, in step b2, a molar ratio of the palladium catalyst to the phosphine ligand is 1:(1-5), preferably 1:(1-2), and more preferably 1:2.

In an optional embodiment of the present disclosure, in step b2, the coupling reaction is carried out under an atmosphere of an inert gas, and the inert gas may be nitrogen, helium, or argon.

In an optional embodiment of the present disclosure, in step b2, a reaction temperature for the coupling reaction may be 65 °C to 75 °C, such as 70 °C.

In an optional embodiment of the present disclosure, in step b2, a reaction time for the coupling reaction may be 10 h to 30 h, preferably 14 h to 18 h.

In an optional embodiment of the present disclosure, in step b2, after the completion of the coupling reaction, a post-treatment step may be further comprised, for example: subjecting a reaction liquid to concentration, acidification, extraction, purification by silica gel column, concentration, co-distillation with methanol, recrystallization, washing, and drying.

The method for preparing compound 1E may further comprise a method for preparing compound 1D, which comprises the following step:
b1) subjecting compound 1C to a coupling reaction as shown below with bis(pinacolato)diboron in the presence of a palladium catalyst and a base in a solvent to give compound 1D:

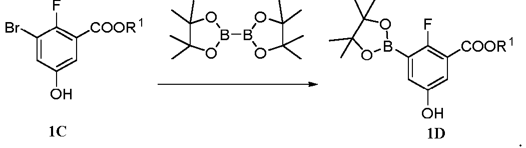

In an optional embodiment of the present disclosure, R¹ is C₁-C₄ alkyl or benzyl, such as methyl or ethyl.

In an optional embodiment of the present disclosure, in step b1, the solvent is selected from water, an alcohol solvent, an aromatic hydrocarbon solvent, an ether solvent, and a mixture of any two or more thereof; preferably, the solvent is an ether solvent. The alcohol solvent is preferably methanol, ethanol, or a mixture thereof. The aromatic hydrocarbon solvent is preferably toluene. The ether solvent is preferably 1,4-dioxane, tetrahydrofuran, or a mixture thereof. In an optional embodiment of the present disclosure, in the method for preparing the compound of formula 1D, the solvent is preferably an ether solvent, more preferably 1,4-dioxane.

In an optional embodiment of the present disclosure, in step b1, the palladium catalyst may be one or more of Pd(OAc)₂, PdCb, Pd(TFA)₂, Pd[O₂C(CH₃)₃]₂, Pd₂(dba)₃, PdBr₂, and Pd(dppf)Cl₂; preferably, the palladium catalyst is Pd(OAc)₂.

In an optional embodiment of the present disclosure, in step b1, the base may be one or more of KHCO₃, NaHCO₃, Na₂CO₃, Ba(OH)₂, K₃PO₄, Cs₂CO₃, K₂CO₃, NaOH, KOH, Et₃N, DIPEA, NaOMe, NaOEt, t-BuOK, t-BuONa, NaH, LiHMDS, NaHMDS, potassium acetate, sodium tert-amylate, n-butyllithium, diethylamine, and dicyclohexylamine; preferably, the base is potassium acetate.

In an optional embodiment of the present disclosure, in step b1, the coupling reaction further comprises a phosphine ligand, and the phosphine ligand may be one or more of PPh₃, SPhos, XPhos, 1,1'-bis(diphenylphosphino)ferrocene, and XantPhos; preferably, the phosphine ligand is XPhos.

In an optional embodiment of the present disclosure, in step b1, R¹ is methyl, the solvent is 1,4-dioxane, the palladium catalyst is Pd(OAc)₂, the base is potassium acetate, and the phosphine ligand Xphos is comprised.

In an optional embodiment of the present disclosure, in step b1, a molar ratio of intermediate 1C to the palladium catalyst is 1:(0.01-0.1), preferably 1:(0.01-0.05), and more preferably 1:0.01.

In an optional embodiment of the present disclosure, in step b1, a molar ratio of intermediate 1C to bis(pinacolato)diboron is 1:(1-5), preferably 1:1.1.

In an optional embodiment of the present disclosure, in step b1, a molar ratio of intermediate 1C to the base is 1:(1-5), preferably 1:(2-3), and more preferably 1:2.5.

In an optional embodiment of the present disclosure, in step b1, a molar ratio of the palladium catalyst to the phosphine ligand is 1:(1-5), preferably 1:(1-2), and more preferably 1:2.

In an optional embodiment of the present disclosure, in step b1, the coupling reaction is carried out under an atmosphere of an inert gas, and the inert gas may be nitrogen, helium, or argon.

In an optional embodiment of the present disclosure, in step b1, a reaction temperature for the coupling reaction may be 75 °C to 85 °C, such as 80 °C.

In an optional embodiment of the present disclosure, in step b1, a reaction time for the coupling reaction may be 2 h to 10 h, preferably 4 h.

In an optional embodiment of the present disclosure, in step b1, after the completion of the coupling reaction, a post-treatment step may be further comprised, for example: subjecting a reaction liquid to filtration through celite, extraction, purification by silica gel column, co-distillation with n-heptane, recrystallization, and drying.

The method for preparing compound 1I may further comprise a method for preparing compound 1H, which comprises the following step:
c3) subjecting compound 1K to a coupling reaction as shown below with 2-bromo-5-methylthiazole in the presence of a palladium catalyst and a base in a solvent to give compound 1H:

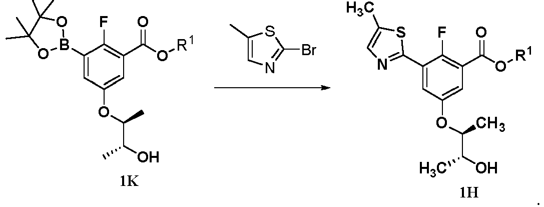

In an optional embodiment of the present disclosure, R¹ is C₁-C₄ alkyl or benzyl, such as methyl or ethyl.

In an optional embodiment of the present disclosure, in step c3, the solvent is selected from water, an alcohol solvent, an aromatic hydrocarbon solvent, an ether solvent, and a mixture of any two or more thereof; preferably, the solvent is a mixture of an ether solvent and water. The alcohol solvent is preferably methanol, ethanol, or a mixture thereof. The aromatic hydrocarbon solvent is preferably toluene. The ether solvent is preferably 1,4-dioxane, tetrahydrofuran, or a mixture thereof. In an optional embodiment of the present disclosure, in the method for preparing the compound of formula 1H, the solvent is preferably a mixture of an ether solvent and water, more preferably a mixture of 1,4-dioxane and water.

In an optional embodiment of the present disclosure, in step c3, the palladium catalyst may be one or more of Pd(OAc)₂, PdCb, Pd(TFA)₂, Pd[O₂C(CH₃)₃]₂, Pd₂(dba)₃, PdBr₂, and Pd(dppf)Cl₂; preferably, the palladium catalyst is Pd(dppf)Cl₂.

In an optional embodiment of the present disclosure, in step c3, the base may be one or more of KHCO₃, NaHCO₃, Na₂CO₃, Ba(OH)₂, K₃PO₄, Cs₂CO₃, K₂CO₃, NaOH, KOH, Et₃N, DIPEA, NaOMe, NaOEt, t-BuOK, t-BuONa, NaH, LiHMDS, NaHMDS, potassium acetate, sodium tert-amylate, n-butyllithium, diethylamine, and dicyclohexylamine; preferably, the base is K₂CO₃.

In an optional embodiment of the present disclosure, in step c3, R¹ is methyl, the solvent is a mixture of 1,4-dioxane and water, the palladium catalyst is Pd(dppf)Cl₂, and the base is K₂CO₃.

In an optional embodiment of the present disclosure, in step c3, a molar ratio of intermediate 1K to the palladium catalyst is 1:(0.01-1), preferably 1:(0.01-0.05), and more preferably 1:0.05.

In an optional embodiment of the present disclosure, in step c3, a molar ratio of intermediate 1K to 2-bromo-5-methylthiazole is 1:(1-5), preferably 1:(1-1.2), and more preferably 1:1.1.

In an optional embodiment of the present disclosure, in step c3, the coupling reaction is carried out under an atmosphere of an inert gas, and the inert gas may be nitrogen, helium, or argon.

In an optional embodiment of the present disclosure, in step c3, a reaction temperature for the coupling reaction may be 85 °C to 95 °C, such as 90 °C.

In an optional embodiment of the present disclosure, in step c3, a reaction time for the coupling reaction may be 1 h to 20 h.

In an optional embodiment of the present disclosure, in step c3, after the completion of the coupling reaction, a post-treatment step may be further comprised, for example: subjecting a reaction liquid to cooling, purification by silica gel column, and concentration to give pure compound 1H.

The method for preparing compound 1H may further comprise a method for preparing compound 1K, which comprises the following step:
c2) subjecting compound 1J to a coupling reaction as shown below with bis(pinacolato)diboron in the presence of a palladium catalyst and a base in a solvent to give compound 1K:

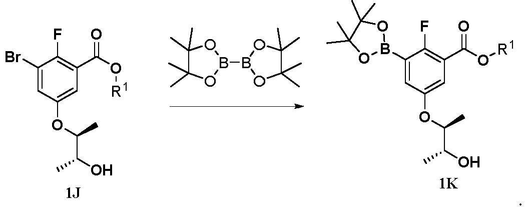

In an optional embodiment of the present disclosure, R¹ is C₁-C₄ alkyl or benzyl, such as methyl or ethyl.

In an optional embodiment of the present disclosure, in step c2, the solvent is selected from water, an alcohol solvent, an aromatic hydrocarbon solvent, an ether solvent, and a mixture of any two or more thereof; preferably, the solvent is an ether solvent. The alcohol solvent is preferably methanol, ethanol, or a mixture thereof; the aromatic hydrocarbon solvent is preferably toluene; the ether solvent is preferably 1,4-dioxane, tetrahydrofuran, or a mixture thereof. In an optional embodiment of the present disclosure, in the method for preparing the compound of formula 1K, the solvent is preferably an ether solvent, more preferably 1,4-dioxane.

In an optional embodiment of the present disclosure, in step c2, the palladium catalyst may be one or more of Pd(OAc)₂, PdCb, Pd(TFA)₂, Pd[O₂C(CH₃)₃]₂, Pd₂(dba)₃, PdBr₂, and Pd(dppf)Cl₂; preferably, the palladium catalyst is Pd(dppf)Cl₂.

In an optional embodiment of the present disclosure, in step c2, the base may be one or more of KHCO₃, NaHCO₃, Na₂CO₃, Ba(OH)₂, K₃PO₄, Cs₂CO₃, K₂CO₃, NaOH, KOH, Et₃N, DIPEA, NaOMe, NaOEt, t-BuOK, t-BuONa, NaH, LiHMDS, NaHMDS, potassium acetate, sodium tert-amylate, n-butyllithium, diethylamine, and dicyclohexylamine; preferably, the base is potassium acetate.

In an optional embodiment of the present disclosure, in step c2, R¹ is methyl, the solvent is 1,4-dioxane, the palladium catalyst is Pd(dppf)Cl₂, and the base is potassium acetate.

In an optional embodiment of the present disclosure, in step c2, a molar ratio of intermediate 1J to the palladium catalyst is 1:(0.01-0.1), preferably 1:(0.01-0.05), and more preferably 1:0.05.

In an optional embodiment of the present disclosure, in step c2, a molar ratio of intermediate 1J to bis(pinacolato)diboron is 1:(1-5), preferably 1:(1-1.2), and more preferably 1:1.1.

In an optional embodiment of the present disclosure, in step c2, the coupling reaction is carried out under an atmosphere of an inert gas, and the inert gas may be nitrogen, helium, or argon.

In an optional embodiment of the present disclosure, in step c2, a reaction temperature for the coupling reaction may be 85 °C to 95 °C, such as 90 °C.

In an optional embodiment of the present disclosure, in step c2, a reaction time for the coupling reaction may be 1 h to 20 h.

In an optional embodiment of the present disclosure, in step c2, after the completion of the coupling reaction, a post-treatment step may be further comprised, for example: subjecting a reaction liquid to cooling, purification by silica gel column, and concentration to give pure compound 1K.

The method for preparing compound 1K may further comprise a method for preparing compound 1J, which comprises the following step:
c1) subjecting compound 1C to a substitution reaction as shown below with compound 1F in the presence of a base in a solvent, followed by acidification with an acid, to give compound 1J:

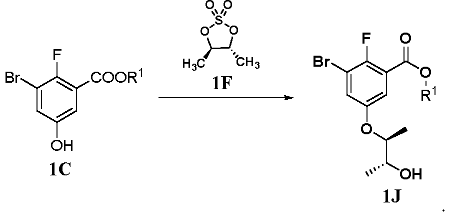

In an optional embodiment of the present disclosure, R¹ is C₁-C₄ alkyl or benzyl, such as methyl or ethyl.

In an optional embodiment of the present disclosure, in step c1, the solvent is selected from water, an alcohol solvent, an aromatic hydrocarbon solvent, an ether solvent, and a mixture of any two or more thereof; preferably, the solvent is an ether solvent. The alcohol solvent is preferably methanol, ethanol, or a mixture thereof; the aromatic hydrocarbon solvent is preferably toluene; the ether solvent is preferably 1,4-dioxane, tetrahydrofuran, or a mixture thereof. In an optional embodiment of the present disclosure, in the method for preparing the compound of formula 1J, the solvent is preferably an ether solvent, more preferably tetrahydrofuran.

In an optional embodiment of the present disclosure, in step c1, the base may be one or more of DIPEA, triethylamine, pyridine, cesium carbonate, potassium carbonate, potassium phosphate, potassium acetate, sodium hydride, sodium hydroxide, and potassium hydroxide; preferably, the base is potassium carbonate.

In an optional embodiment of the present disclosure, in step c1, the acid may be sulfuric acid, preferably a 20%-75% aqueous sulfuric acid solution, and more preferably a 20% aqueous sulfuric acid solution.

In an optional embodiment of the present disclosure, in step c1, a molar ratio of compound 1C to the acid is 1:(1-5), preferably 1:2.

In an optional embodiment of the present disclosure, in step c1, the solvent is tetrahydrofuran, the base is potassium carbonate, and the acid is a 20% aqueous sulfuric acid solution.

In an optional embodiment of the present disclosure, in step c1, a molar ratio of compound 1C to compound 1F is 1:(1-5), preferably 1:(1.1-1.3), and more preferably 1:1.2.

In an optional embodiment of the present disclosure, in step c1, a molar ratio of compound 1C to the base is 1:(1-5), preferably 1:(1-2), and more preferably 1:(1.5).

In an optional embodiment of the present disclosure, in step c1, a reaction temperature for the substitution reaction may be 45 °C to 55 °C, preferably 50 °C.

In an optional embodiment of the present disclosure, in step c1, a temperature for the acidification is 50 °C to 70 °C, preferably 55 °C to 65 °C.

In an optional embodiment of the present disclosure, in step c1, a reaction time for the substitution reaction may be 1 h to 20 h.

In an optional embodiment of the present disclosure, in step c1, after the completion of the substitution reaction, a post-treatment step may be further comprised, for example: subjecting a reaction liquid to cooling, acidification, extraction, washing, drying, and purification by silica gel column to give pure compound 1H.

In an optional embodiment of the present disclosure, step c1 comprises the following steps: adding compound 1C, compound 1F, and a base to a solvent for a first-stage reaction, and then adding an acid to the reaction system for acidification to give compound 1H.

The method for preparing compound 1D or compound 1J may further comprise a method for preparing compound 1C, which comprises the following step:
d2) subjecting compound 1B to an esterification reaction as shown below with R¹-OH in the presence of a catalyst in a solvent to give compound 1C:

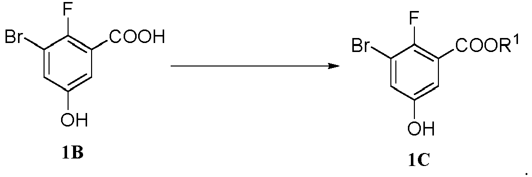

In an optional embodiment of the present disclosure, in step d2, the solvent is selected from water, an alcohol solvent, an aromatic hydrocarbon solvent, an ether solvent, and a mixture of any two or more thereof; preferably, the solvent is an alcohol solvent. The alcohol solvent is preferably methanol, ethanol, or a mixture thereof. The aromatic hydrocarbon solvent is preferably toluene. The ether solvent is preferably 1,4-dioxane, tetrahydrofuran, or a mixture thereof. In an optional embodiment of the present disclosure, in the method for preparing the compound of formula 1C, the solvent is preferably an alcohol solvent, more preferably methanol.

In an optional embodiment of the present disclosure, in step d2, R¹ in R¹-OH is C₁-C₄ alkyl or benzyl, such as methyl or ethyl.

In an optional embodiment of the present disclosure, in step d2, the catalyst may be sulfuric acid, phosphoric acid, thionyl chloride, or acetyl chloride; preferably, the catalyst is acetyl chloride.

In an optional embodiment of the present disclosure, in step d2, the solvent is methanol, the catalyst is acetyl chloride, and R¹-OH is methanol.

In an optional embodiment of the present disclosure, in step d2, a molar ratio of compound 1B to the catalyst is 1:(0.1-1), preferably 1:(0.5-1), and more preferably 1:1.

In an optional embodiment of the present disclosure, in step d2, a reaction temperature for the esterification reaction may be 55 °C to 65 °C, such as 60 °C.

In an optional embodiment of the present disclosure, in step d2, a reaction time for the esterification reaction may be 1 h to 20 h, such as 4 h.

In an optional embodiment of the present disclosure, in step d2, after the completion of the esterification reaction, a post-treatment step may be further comprised, for example: subjecting a reaction liquid to cooling, slow addition to ice water to quench the reaction, centrifugation, and drying to give pure compound 1C.

The method for preparing compound 1C may further comprise a method for preparing compound 1B, which comprises the following step:
d1) subjecting compound 1A to a substitution reaction as shown below with water in the presence of a cuprous catalyst and an inorganic base in a solvent to give compound 1B:

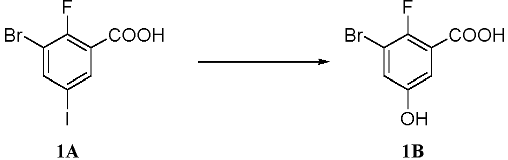

In an optional embodiment of the present disclosure, in step d1, the solvent is selected from water, an alcohol solvent, an aromatic hydrocarbon solvent, an ether solvent, and a mixture of any two or more thereof; preferably, the solvent is water. The alcohol solvent is preferably methanol, ethanol, or a mixture thereof. The aromatic hydrocarbon solvent is preferably toluene. The ether solvent is preferably 1,4-dioxane, tetrahydrofuran, or a mixture thereof. In an optional embodiment of the present disclosure, in the method for preparing the compound of formula 1B, the solvent is preferably water.

In an optional embodiment of the present disclosure, in step d1, the cuprous catalyst may be one or more of copper(I) bromide, copper(I) chloride, copper(I) iodide, and copper(I) oxide; preferably, the cuprous catalyst is copper(I) oxide. In an optional embodiment of the present disclosure, in step d1, the inorganic base may be one or more of KHCO₃, NaHCO₃, Na₂CO₃, Ba(OH)₂, K₃PO₄, Cs₂CO₃, K₂CO₃, NaOH, KOH, and NaH; preferably, the base is NaOH.

In an optional embodiment of the present disclosure, in step d1, the solvent is water, the cuprous catalyst is copper(I) oxide, and the inorganic base is NaOH.

In an optional embodiment of the present disclosure, in step d1, a molar ratio of intermediate 1A to the cuprous catalyst is 1:(0.1-2), preferably 1:0.2.

In an optional embodiment of the present disclosure, in step d1, a molar ratio of intermediate 1A to the inorganic base is 1:(1-5), preferably 1:(3-5), and more preferably 1:5.

In an optional embodiment of the present disclosure, in step d1, a reaction temperature for the substitution reaction may be 90 °C to 100 °C, such as 95 °C.

In an optional embodiment of the present disclosure, in step d1, a reaction time for the substitution reaction may be 1 h to 20 h, such as 10 h.

In an optional embodiment of the present disclosure, in step d1, after the completion of the substitution reaction, a post-treatment step may be further comprised, for example: subjecting a reaction liquid to cooling, filtration through celite, washing, pH adjustment with diluted hydrochloric acid until pH = 1 to 3, extraction, and concentration to give pure compound 1H.

### Beneficial Effects

The positive progress effects of the present disclosure are as follows: Provided is a novel method for preparing 2-fluoro-5-(((2S,3R)-3-hydroxybutan-2-yl)oxy)-3-(5-methylthiazol-2-yl)-N-((R)-1-(2-(trifluoromethyl)pyrimidin-5-yl)ethyl)benzamide and an intermediate thereof. The preparation method is simple to operate, facilitates quality control, achieves high yield, and is suitable for industrial production.

### Definitions and Description

In the present disclosure, the term "C₁-C₄ alkyl" represents a saturated linear or branched alkyl group with 1-4 carbon atoms, such as methyl, ethyl, n-propyl, isopropyl, n-butyl, isobutyl, tert-butyl, and the like, in particular methyl or ethyl.

### Abbreviation

| | |
|---|---|
| HOBt | 1-Hydroxybenzotriazole |
| EDCI | 1-Ethyl-3(3-dimethylpropylamine)carbodiimide |
| EA | Ethyl acetate |
| T₃P | 1-Propylphosphonic anhydride |
| PyBOP | (Benzotriazol-1-yloxy)tripyrrolidinophosphonium hexafluorophosphate |
| DCC | N,N'-Dicyclohexylcarbodiimide |
| CDI | N,N'-Carbonyldiimidazole |
| HATU | O-(7-Azabenzotriazol-1-yl)-N,N,N',N'-tetramethyluronium hexafluorophosphate |
| DIPEA | N,N-Diisopropylethylamine |
| SPhos | 2-Dicyclohexylphosphino-2',6'-dimethoxy-1,1'-biphenyl |
| XPhos | 2-Dicyclohexylphosphino-2',4',6'-triisopropylbiphenyl |
| XantPhos | 4,5-Bis(diphenylphosphino)-9,9-dimethylxanthene |
| PPh3 | Triphenylphosphine |
| LiHMDS | Lithium bis(trimethylsilyl)amide |
| NaHMDS | Sodium bis(trimethylsilyl)amide |

The preferred conditions described above may be combined arbitrarily to give preferred embodiments of the present disclosure without departing from the general knowledge in the art.

The reagents and starting materials used in the present disclosure are commercially available.

### DETAILED DESCRIPTION

The technical solutions of the present disclosure will be further explained in detail by the description of the following specific examples. It should be understood that the following examples are merely exemplary illustrations and explanations of the present disclosure, and should not be construed as limiting the protection scope of the present disclosure. All techniques implemented based on the content of the present disclosure described above are encompassed within the protection scope of the present disclosure.

Unless otherwise stated, the starting materials and reagents used in the following examples are all commercially available products or can be prepared by using known methods.

### Steps 1 and 2: Preparation of intermediate 1C

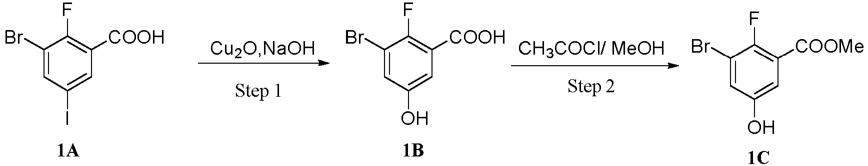

Water (614.0 kg) was transferred to a clean 2000 L reaction kettle, followed by the addition of sodium hydroxide (50.2 kg), compound 1A (86.55 kg), and copper(I) oxide (7.13 kg) in 5 batches. The mixture was warmed to 95 °C and reacted at a maintained temperature for 10 h. A sample was collected and analyzed by HPLC, which indicated the complete consumption of the starting materials.

The system was cooled to 25 °C, filtered through celite (22.5 kg), washed with water (50.0 kg), and adjusted to pH = 1 to 3 with an aqueous solution of diluted hydrochloric acid (prepared by pouring concentrated hydrochloric acid (185.0 kg) into water (400.0 kg)). Ethyl acetate (615 kg) was added for phase separation, and the aqueous phase was added with ethyl acetate (353.0 kg) for extraction. The organic phases were combined, and the aqueous phase was disposed of as a waste liquid after COD testing confirmed compliance. The organic phase was concentrated under vacuum (internal temperature ≤ 55 °C, and vacuum ≤ -0.07 MPa) until no significant distillate was observed, followed by co-distillation twice with methanol (320.0 kg × 2). A sample was collected and analyzed by HPLC (purity: qualified). Methanol (120 kg) was transferred into the system, and the product was stored in methanol for feeding in the next step, with a total weight of 190.0 kg, a content of 29.3%, and a yield of 94.1%.

The solution of intermediate 1B in methanol (280.0 kg) from the previous step was added to a clean 2000 L reaction kettle and cooled to 0-10 °C. Acetyl chloride (36.0 kg) was added dropwise (with the temperature controlled to be ≤40 °C during the addition), and the mixture was warmed to 60 °C and reacted for 4 h. A sample was collected and analyzed by HPLC, which indicated the completion of the reaction. The reaction was terminated.

The system was cooled to 25 °C, and ice water (275.0 kg) was added. After 1 h of stirring, the system was centrifuged and washed with a 33.3% methanol-water solution (185 kg) to give wet intermediate 1C. A sample was collected, analyzed by HPLC (purity: qualified), and dried in a vacuum drying oven for 20 h to give dry intermediate 1C (53.6 kg, yield: 91.6%).

LC-MS, M/Z (ESI): 248.9 [M+H]⁺.

### Step 3: Preparation of intermediate 1D

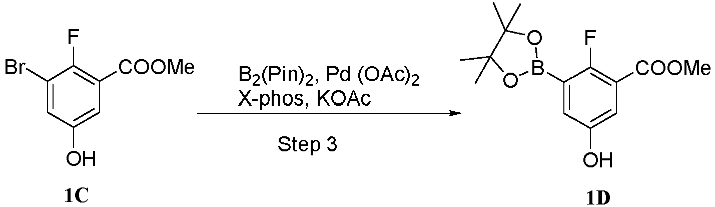

Dioxane (560.0 kg), intermediate 1C (53.5 kg), bis(pinacolato)diboron (60.0 kg), Pd(OAc)₂ (0.48 kg), Xphos (2.0 kg), and potassium acetate (52.0 kg) were added to a dry and clean 2000 L reaction kettle. The system was purged 3 times with argon, warmed to 80 °C, and reacted at a maintained temperature for 4 h. A sample was collected and analyzed by HPLC, which confirmed the completion of the reaction.

The system was cooled to 25 °C, filtered through celite (25.0 kg), washed with ethyl acetate (150.0 kg), and concentrated. Ethyl acetate (480.0 kg) and water (266 kg) were added for phase separation. The organic phase was passed through silica gel column, washed with ethyl acetate (94.0 kg), concentrated, co-distilled twice with n-heptane (75.0 kg × 4), crystallized once, centrifuged, and then washed once more to give wet intermediate 1D. A sample was collected, analyzed by HPLC (purity: qualified), placed in a vacuum drying oven, and dried under vacuum at 55 °C to 60 °C for 16 h to give dry intermediate 1D (56.5 kg, yield: 89.1%) as an off-white solid.

LC-MS, M/Z (ESI): 297.1 [M+H]⁺.

### Step 4: Preparation of intermediate 1E

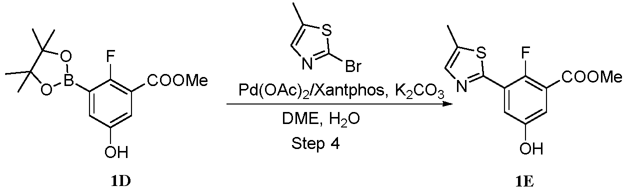

Ethylene glycol dimethyl ether (480.0 kg), intermediate 1D (56.5 kg), 2-bromo-5-methylthiazole (32.8 kg), a 47% aqueous potassium carbonate solution (107 kg), Pd(OAc)₂ (0.83 kg), and Xantphos (4.2 kg) were added to a clean 2000 L reaction kettle. The system was purged three times with argon, warmed to 70 °C, and reacted for 14-18 h. A sample was collected and analyzed by HPLC, which confirmed the completion of the reaction.

The internal temperature was reduced to 40 °C, and the system was concentrated under vacuum (jacket temperature ≤ 55 °C, and vacuum ≤ -0.07 MPa) until no significant distillate was observed and cooled to 25 °C. An aqueous citric acid solution (prepared by pouring citric acid (72 kg) into water (270 kg)) was added, followed by the addition of ethyl acetate (540.0 kg) for phase separation. The aqueous phase was extracted with ethyl acetate (110.0 kg). The organic phases were combined and filtered through silica gel (40.0 kg). The filter cake was washed with ethyl acetate (110.0 kg), concentrated under vacuum (jacket temperature ≤ 55 °C, and vacuum ≤ -0.07 MPa) until no significant distillate was observed, and co-distilled twice with methanol (79.0 kg × 2) until no distillate was observed. Methanol (100.0 kg) was added, and the mixture was crystallized at 5 °C for 2 h, centrifuged, and washed with methanol (79 kg) to give wet intermediate 1E. A sample was collected, analyzed by HPLC (purity: qualified), and then dried in a vacuum drying oven for 16 h to give dry intermediate 1E (33.4 kg, yield: 65.6%).

LC-MS, M/Z (ESI): 268.2 [M+H]⁺.

### Step 5: Preparation of intermediate 1H

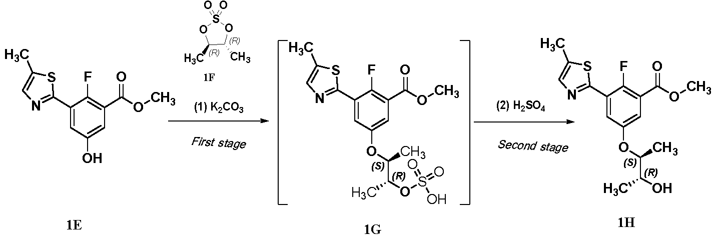

Sequentially, tetrahydrofuran (29.5 kg) was added to and intermediate 1E (4.15 kg), N,N-dimethylformamide (3.92 kg), compound 1F (3.07 kg), and anhydrous potassium carbonate (fine powder, 4.29 kg) were added under stirring to a 100 L glass reaction kettle. The system was warmed to 65°C to 70 °C and reacted at a maintained temperature for 12 h. Then, a sample was collected and monitored by TLC until the spot of the starting material, intermediate 1E, was invisible to the naked eye. After the first-stage reaction was completed, the reaction liquid was cooled to 0 °C to 20 °C, and a prepared 20% aqueous sulfuric acid solution (15.25 kg) was slowly added dropwise. After the dropwise addition was completed, the mixture was warmed to 55 °C to 65 °C. After 3 h of reaction, a sample was collected and monitored by TLC until the spot of intermediate 1G was invisible to the naked eye.

The mixture was cooled to 10 °C to 20 °C and allowed to stand for phase separation. The upper organic layer was collected. Ethyl acetate (18.7 kg) was added to the lower aqueous phase, and the mixture was stirred and also allowed to stand for phase separation. The upper organic layer was collected again. The two organic phases were combined, added with a saturated aqueous sodium bicarbonate solution for washing until the aqueous layer achieved pH = 7 to 8 (based on the pH value), then added with purified water (20.75 kg) for washing once. The organic layer was concentrated under reduced pressure to dryness at 50 °C to 60 °C to give crude intermediate 1H (about 6.7 kg). Methanol (6.6 kg) was added to a dry and clean 50 L glass reaction kettle, followed by the addition of the crude intermediate 1H (about 6.7 kg) that was concentrated to dryness as described above. The mixture was warmed to 50 °C to 70 °C and stirred to dissolve completely. Purified water (8.3 kg) was slowly added dropwise at 60 °C to 70 °C for about 1.0 h. After the dropwise addition was completed, heating was stopped, and the mixture was cooled to 10 °C to 20 °C, crystallized for 1 h to 2 h, centrifuged, and rinsed once with a prepared mixed solvent of methanol/purified water (3.0 kg/3.75 kg). The filter cake was collected and weighed to give wet intermediate 1H (5.455 kg). The wet product was tested for weight loss on drying, and the weight of the dry product was then directly calculated for use in the next step.

LC-MS, M/Z (ESI): 340.1 [M+H]⁺.

### Step 6: Preparation of 1I

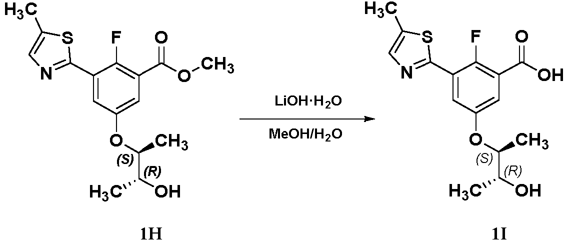

Methanol (32.1 kg) and wet intermediate 1H (5.37 kg) were added to a 100 L glass reaction kettle, and the temperature was maintained at 10 °C to 30 °C. A prepared 13% aqueous LiOH•H₂O solution (10.31 kg) was slowly added dropwise. The mixture was reacted at 10 °C to 30 °C for 2 h, and then a sample was collected and monitored by TLC until intermediate 1H disappeared.

After the reaction was completed, the system was cooled to 0 °C to 20 °C, and then 4 M hydrochloric acid was added in batches. First, the pH was adjusted to 4 (about 6.5 kg of hydrochloric acid was used), and crystallization was performed at a maintained temperature for 1 h to 2 h to precipitate a large amount of white solid. The remaining 4 M hydrochloric acid was then slowly added dropwise to adjust the pH to 2 to 3, and crystallization was performed under stirring at a maintained temperature of 10 °C to 20 °C for 1 h to 2 h. After centrifugation and three rinses with purified water (10.0 kg × 3), the wet filter cake was collected and blow-dried at 65±5 °C for not less than 8 h to give dry intermediate 1I (4.345 kg).

LC-MS, M/Z (ESI): 324.1 [M-H]⁺.

### Step 7: Preparation of finished product

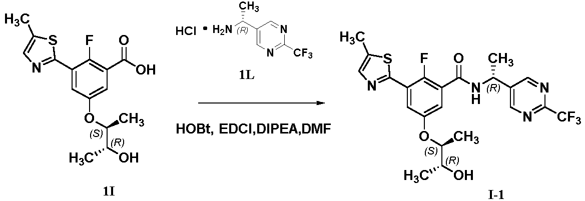

Under a nitrogen atmosphere, DMF (20.48 kg) and intermediate 1I (4.32 kg) were added to a 100 L glass reaction kettle, and the system was cooled to 10 °C to 25 °C. HOBt (2.68 kg), EDCI (3.80 kg), and compound 1L (3.63 kg) were added sequentially under stirring. The mixture was cooled, and DIPEA (5.14 kg) was slowly added dropwise. The temperature of the reaction liquid was maintained at 5 °C to 20 °C. After the dropwise addition was completed, the reaction was carried out at 10 °C to 30 °C. After 2 h, a sample was collected and monitored by TLC until intermediate 1I disappeared. The reaction was stopped.

The reaction liquid was added with ethyl acetate (77.76 kg) and then added with a 6% aqueous sodium bicarbonate solution (64.8 kg × 2) for washing twice, and the organic phase was collected. Washing was performed once more with a 0.2 M aqueous hydrochloric acid solution until the pH = 3 to 4, and the organic phase was collected. Washing was performed twice with purified water (43.2 kg × 2) until the pH was neutral. The organic phase was collected. The organic layer was concentrated under vacuum at 50 °C to 60 °C until a small amount of solvent remained. Then, n-heptane (40.0 kg) was added in batches to a rotary evaporation flask. The system was transferred into a turnover bucket, stirred at 10 °C to 30 °C for 0.5-2 h, and centrifuged. The filter cake was dried in a blast air oven at 55±5 °C for not less than 8 h to give an off-white to yellow solid. Weighing was performed to give dry compound I-1 (5.64 kg, yield: 98%).

Absolute ethanol (8.68 kg) was added to a 50 L spherical glass reaction kettle, followed by the addition of compound I-1 (2.80 kg) under stirring. The mixture was heated to 60-80 °C to dissolve completely and filtered. The filtrate was collected. The filtrate was heated to dissolve completely again, and then n-heptane (13.44 kg) was slowly added dropwise to the reaction kettle at 60 °C to 80 °C for about 1.0 h to precipitate a solid. The system was slowly cooled to 15 °C to 25 °C, stirred for 1 h to 3 h, and centrifuged. The filter cake was collected and dried in a vacuum drying oven at 55±5 °C for not less than 8 h to give a finished product (2.53 kg).
LC-MS, M/Z (ESI): 499.2 [M+H]⁺.
¹H NMR (400 MHz, Chloroform-d) δ9.20 (1H, d, J = 7.2 Hz), 9.11 (1H, s), 7.74 (1H, dd, J = 5.6, 3.2 Hz), 7.70 (1H, dd, J = 2.3, 1.2 Hz), 7.19 (1H, dd, J = 5.3, 3.2 Hz), 5.26 (1H, p, J = 7.0 Hz), 4.80 (1H, d, J = 5.1 Hz), 4.29 (1H, qd, J = 6.1, 4.4 Hz), 3.71 (1H, m), 2.50 (3H, d, J = 1.0 Hz), 1.55 (3H, d, J = 7.1 Hz), 1.20 (3H, d, J = 6.2 Hz), 1.11 (3H, d, J = 6.4 Hz)

### Example 2: Preparation of Intermediate 1H

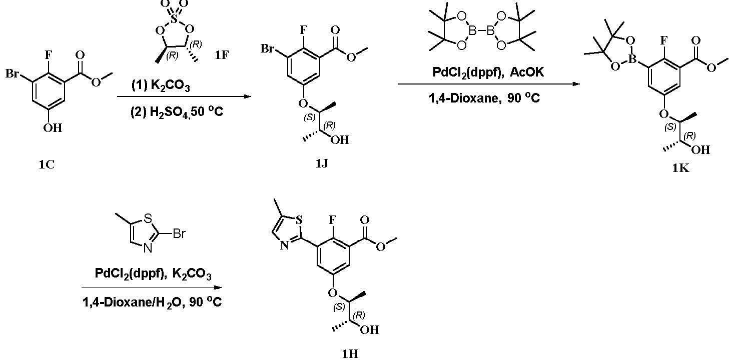

### Step 1: Synthesis of methyl 3-bromo-2-fluoro-5-(((2S,3R)-3-hydroxybutan-2-yl)oxy)benzoate (1J)

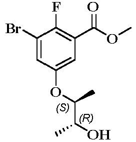

Methyl 3-bromo-2-fluoro-5-hydroxybenzoate (1C) (30 g, 120 mmol) was placed in a round-bottom flask, and compound 1F (22 g, 145 mmol), potassium carbonate (24.97 g, 181 mmol), and tetrahydrofuran (30 mL) were added. The mixture was refluxed overnight. After the starting materials were completely reacted as detected by TLC, the reaction liquid was cooled to room temperature. 20% H₂SO₄ (300 mL) was added to the reaction liquid, and the mixture was heated at 50 °C for 6 h. After the starting materials were completely consumed as detected by LCMS, the reaction liquid was cooled to room temperature. EA was added for extraction. The organic phase was collected, washed with a saturated sodium bicarbonate solution (200 mL), dried over anhydrous sodium sulfate, and separated and purified by silica gel column (petroleum ether:ethyl acetate (V/V) = 10:1 to 3:1) to give a yellow oily liquid (20.0 g, yield: 77%).

LC-MS, M/Z (ESI): 321.0 [M+H]⁺.

### Step 2: Synthesis of methyl 2-fluoro-5-(((2S,3R)-3-hydroxybutan-2-yl)oxy)-3-(4,4,5,5-tetramethyl-1,3,2-dioxaborolan-2-yl)benzoate (1K)

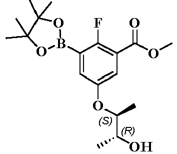

Methyl 3-bromo-2-fluoro-5-(((2S,3R)-3-hydroxybutan-2-yl)oxy)benzoate (50 g, 156 mmol) was placed in a three-necked flask containing 1,4-dioxane (500 mL), and bis(pinacolato)diboron (43.5 g, 171 mmol), Pd(dppf)Cl₂ (5.70 g, 7.78 mmol), and potassium acetate (45.8 g, 467 mmol) were added. The mixture was purged three times with nitrogen and reacted at 90 °C overnight under a nitrogen atmosphere. After the starting materials were completely consumed as detected by TLC, the reaction liquid was cooled to room temperature, concentrated, and directly separated and purified by silica gel column (100% ethyl acetate). Concentration was performed to give an oily liquid, which was directly used in the next reaction.

### Step 3: Synthesis of methyl 2-fluoro-5-(((2S,3R)-3-hydroxybutan-2-yl)oxy)-3-(5-methylthiazol-2-yl)benzoate (1H)

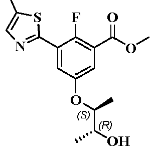

Methyl 2-fluoro-5-(((2S,3R)-3-hydroxybutan-2-yl)oxy)-3-(4,4,5,5-tetramethyl-1,3,2-dioxaborolan-2-yl)benzoate (28.7 g, 78 mmol) was added to a solution of 1,4-dioxane (300 mL) and water (30 mL), and Pd(dppf)Cl₂ (2.58 g, 3.9 mmol), 2-bromo-5-methylthiazole (15.27 g, 86 mmol), and potassium carbonate (21.5 g, 156 mmol) were added. The mixture was purged three times with nitrogen and reacted at 90 °C overnight under a nitrogen atmosphere. After the starting materials were completely consumed as detected by TLC, the reaction liquid was cooled to room temperature, concentrated, and directly separated and purified by silica gel column (petroleum ether:ethyl acetate (V/N) = 10:1 to 3:1) to give a yellow oily liquid (20.0 g, yield: 76%).

LC-MS, M/Z (ESI): 340.1 [M+H]⁺.

The embodiments of the technical solutions of the present disclosure have been described above by way of example. It should be understood that the protection scope of the present disclosure is not limited to the embodiments described above. Any modification, equivalent replacement, improvement, and the like made by those skilled in the art without departing from the spirit and principle of the present disclosure shall fall within the protection scope of the claims of the present application.

## Claims

1. A method for preparing compound I-1, wherein the method comprises the following step:
a2) subjecting compound 1I and compound 1L to a condensation reaction as shown below under the action of a condensing agent and a base in a solvent to give compound I-1;

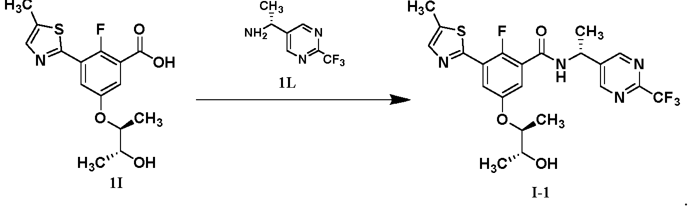

2. The preparation method according to claim 1, wherein
the organic solvent may be selected from water, a chlorinated alkane solvent, an ether solvent, an amide solvent, an arene solvent, an ester solvent, and a mixture of any two or more thereof; the organic solvent is preferably an amide solvent, more preferably N,N-dimethylformamide;
and/or compound 1L is present as a free base or a salt thereof, such as a hydrochloride salt of compound 1L;
and/or the condensing agent may be one or more of HOBt/EDCI, T₃P, PyBOP, DCC, CDI, or HATU; preferably, the condensing agent is HOBt/EDCI,
and/or the base may be DIPEA, sodium bicarbonate, potassium carbonate, cesium carbonate, triethylamine, or pyridine; preferably, the base is DIPEA;
and/or a molar ratio of intermediate 1I to the condensing agent may be 1:(1-5), preferably 1:(1.5-2), and more preferably 1:1.5;
and/or a molar ratio of intermediate 1I to the base may be 1:(1-5), preferably 1:(2-4), and more preferably 1:3;
and/or a molar ratio of intermediate 1I to intermediate 1L may be 1:(1-4), preferably 1:(1-3), and more preferably 1:1.2;
and/or the condensation reaction is carried out under an atmosphere of an inert gas, and the inert gas may be nitrogen, helium, or argon;
and/or after the completion of the condensation reaction, a post-treatment step may be further comprised, for example: subjecting a reaction liquid to extraction, centrifugation, drying, recrystallization, centrifugation, and drying.

3. The preparation method according to any one of claims 1-2, wherein the preparation method may further comprise a method for preparing compound 1I comprising the following step:
a1) subjecting compound 1H to a hydrolysis reaction as shown below in the presence of a basic substance in a solvent to give compound 1I;

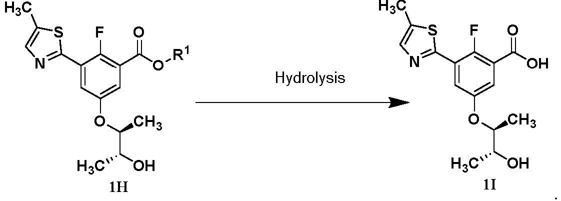

4. The preparation method according to claim 3, wherein
R¹ is C₁-C₄ alkyl or benzyl, such as methyl or ethyl;
and/or the solvent is an alcohol solvent, water, or a mixed solvent of an alcohol solvent and water, preferably a mixed solvent of an alcohol solvent and water, and more preferably a mixed solvent of methanol and water; and/or the basic substance may be one or more of triethylamine, potassium tert-butoxide, sodium tert-butoxide, lithium carbonate, potassium carbonate, sodium carbonate, sodium hydroxide, lithium hydroxide, or potassium hydroxide; preferably, the basic substance is lithium hydroxide;
and/or a molar ratio of compound 1H to the basic substance is 1:(1-5), preferably 1:2;
and/or a reaction time for the hydrolysis reaction may be 1 h to 20 h, such as 1 h to 2 h;
and/or after the completion of the hydrolysis reaction, a post-treatment step may be further comprised, for example: subjecting a reaction liquid to cooling, acidification, centrifugation, washing, and drying to give pure compound 1I.

5. The preparation method according to any one of claims 3-4, wherein the preparation method may further comprise a method for preparing compound 1H comprising the following step:
b3) subjecting compound 1E to a substitution reaction as shown below with 1F in the presence of a base in a solvent, followed by acidification with an acid, to give compound 1H:

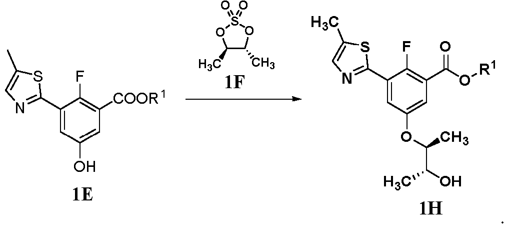

6. The preparation method according to claim 5, wherein
R¹ is C₁-C₄ alkyl or benzyl, such as methyl or ethyl;
and/or the solvent is an alcohol solvent, an aromatic hydrocarbon solvent, an ether solvent, an amide solvent, or a mixture of any two or more thereof, preferably a mixture of an ether solvent and an amide solvent, and more preferably a mixture of tetrahydrofuran and N,N-dimethylformamide;
and/or the base may be one or more of DIPEA, triethylamine, pyridine, cesium carbonate, potassium carbonate, potassium phosphate, potassium acetate, sodium hydride, sodium hydroxide, and potassium hydroxide; preferably, the base is potassium carbonate;
and/or the acid may be sulfuric acid, preferably a 20%-75% aqueous sulfuric acid solution, and more preferably a 20% aqueous sulfuric acid solution;
and/or a molar ratio of compound 1E to compound 1F is 1:(1-5), preferably 1:(1.3-1.5), and more preferably 1:1.3; and/or a molar ratio of compound 1E to the base is 1:(1-5), preferably 1:2;
and/or a mixing volume ratio of tetrahydrofuran to N,N-dimethylformamide is (1-10):1;
and/or a molar ratio of compound 1E to the acid is 1:(1-5), preferably 1:2; and/or a reaction temperature for the substitution reaction may be 60 °C to 75 °C, preferably 65 °C to 70 °C;
and/or a temperature for the acidification is 50 °C to 70 °C, preferably 55 °C to 65 °C;
and/or after the completion of the substitution reaction, a post-treatment step may be further comprised, for example:
subjecting a reaction liquid to cooling, phase separation by standing, extraction, washing, concentration, recrystallization, centrifugation, and washing to give wet compound 1H;
preferably, step b3 comprises the following steps: adding compound 1E, compound 1F, and a base to a solvent for a first-stage reaction, and then adding an acid to the reaction system for acidification to give compound 1H.

7. The preparation method according to any one of claims 5-6, wherein the preparation method may further comprise a method for preparing compound 1E comprising the following step:
b2) subjecting compound 1D to a coupling reaction as shown below with 2-bromo-5-methylthiazole in the presence of a palladium catalyst and a base in a solvent to give compound 1E:

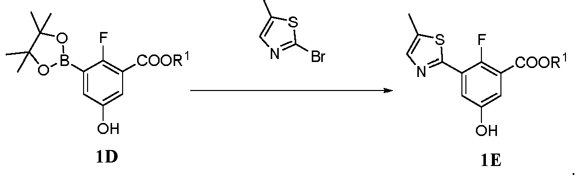

8. The preparation method according to claim 7, wherein
R¹ is C₁-C₄ alkyl or benzyl, such as methyl or ethyl;
and/or the solvent is water, an alcohol solvent, an aromatic hydrocarbon solvent, an ether solvent, or a mixture of any two or more thereof, preferably an ether solvent, and more preferably ethylene glycol dimethyl ether;
and/or the palladium catalyst may be one or more of Pd(OAc)₂, PdCb, Pd(TFA)₂, Pd[O₂C(CH₃)₃]₂, Pd₂(dba)₃, PdBr₂, and Pd(dppf)Cl₂; preferably, the palladium catalyst is Pd(OAc)₂;
and/or the base may be one or more of KHCO₃, NaHCO₃, Na₂CO₃, Ba(OH)₂, K₃PO₄, Cs₂CO₃, K₂CO₃, NaOH, KOH, Et₃N, DIPEA, NaOMe, NaOEt, t-BuOK, t-BuONa, NaH, LiHMDS, NaHMDS, potassium acetate, sodium tert-amylate, n-butyllithium, diethylamine, and dicyclohexylamine; preferably, the base is K₂CO₃;
and/or the coupling reaction further comprises a phosphine ligand, and the phosphine ligand may be one or more of PPh₃, SPhos, XPhos, 1,1'-bis(diphenylphosphino)ferrocene, and XantPhos; preferably, the phosphine ligand is XantPhos;
and/or a molar ratio of intermediate 1D to the palladium catalyst is 1:(0.01-0.1), preferably 1:(0.01-0.05), and more preferably 1:0.02;
and/or a molar ratio of intermediate 1D to 2-bromo-5-methylthiazole is 1:(0.5-5), preferably 1:1;
and/or a molar ratio of intermediate 1D to the base is 1:(1-5), preferably 1:(1.5-2), and more preferably 1:1.9;
and/or a molar ratio of the palladium catalyst to the phosphine ligand is 1:(1-5), preferably 1:(1-2), and more preferably 1:2;
and/or the coupling reaction is carried out under an atmosphere of an inert gas, and the inert gas may be nitrogen, helium, or argon;
and/or a reaction temperature for the coupling reaction may be 65 °C to 75 °C, such as 70 °C;
and/or a reaction time for the coupling reaction may be 10 h to 30 h, preferably 14 h to 18 h;
and/or after the completion of the coupling reaction, a post-treatment step may be further comprised, for example: subjecting a reaction liquid to concentration, acidification, extraction, purification by silica gel column, concentration, co-distillation with methanol, recrystallization, washing, and drying.

9. The preparation method according to any one of claims 7-8, wherein the preparation method may further comprise a method for preparing compound 1D comprising the following step:
b1) subjecting compound 1C to a coupling reaction as shown below with bis(pinacolato)diboron in the presence of a palladium catalyst and a base in a solvent to give compound 1D:

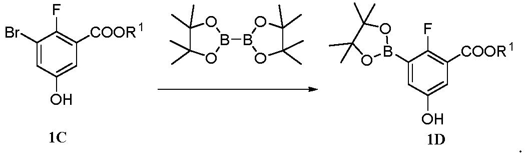

10. The preparation method according to claim 9, wherein
R¹ is C₁-C₄ alkyl or benzyl, such as methyl or ethyl;
and/or the solvent is water, an alcohol solvent, an aromatic hydrocarbon solvent, an ether solvent, or a mixture of any two or more thereof, preferably an ether solvent, and more preferably 1,4-dioxane;
and/or the palladium catalyst may be one or more of Pd(OAc)₂, PdCb, Pd(TFA)₂, Pd[O₂C(CH₃)₃]₂, Pd₂(dba)₃, PdBr₂, and Pd(dppf)Cl₂; preferably, the palladium catalyst is Pd(OAc)₂;
and/or the base may be one or more of KHCO₃, NaHCO₃, Na₂CO₃, Ba(OH)₂, K₃PO₄, Cs₂CO₃, K₂CO₃, NaOH, KOH, Et₃N, DIPEA, NaOMe, NaOEt, t-BuOK, t-BuONa, NaH, LiHMDS, NaHMDS, potassium acetate, sodium tert-amylate, n-butyllithium, diethylamine, and dicyclohexylamine; preferably, the base is potassium acetate;
and/or the coupling reaction further comprises a phosphine ligand, and the phosphine ligand may be one or more of PPh₃, SPhos, XPhos, 1,1'-bis(diphenylphosphino)ferrocene, and XantPhos; preferably, the phosphine ligand is XPhos;
and/or a molar ratio of intermediate 1C to the palladium catalyst is 1:(0.01-0.1), preferably 1:(0.01-0.05), and more preferably 1:0.01;
and/or a molar ratio of intermediate 1C to bis(pinacolato)diboron is 1:(1-5), preferably 1:1.1;
and/or a molar ratio of intermediate 1C to the base is 1:(1-5), preferably 1:(2-3), and more preferably 1:2.5;
and/or a molar ratio of the palladium catalyst to the phosphine ligand is 1:(1-5), preferably 1:(1-2), and more preferably 1:2;
and/or the coupling reaction is carried out under an atmosphere of an inert gas, and the inert gas may be nitrogen, helium, or argon;
and/or a reaction temperature for the coupling reaction may be 75 °C to 85 °C, such as 80 °C;
and/or a reaction time for the coupling reaction may be 2 h to 10 h, preferably 4 h;
and/or after the completion of the coupling reaction, a post-treatment step may be further comprised, for example: subjecting a reaction liquid to filtration through celite, extraction, purification by silica gel column, co-distillation with n-heptane, recrystallization, and drying.

11. The preparation method according to any one of claims 1-2, wherein the preparation method may further comprise a method for preparing compound 1H comprising the following step:
c3) subjecting compound 1K to a coupling reaction as shown below with 2-bromo-5-methylthiazole in the presence of a palladium catalyst and a base in a solvent to give compound 1H:

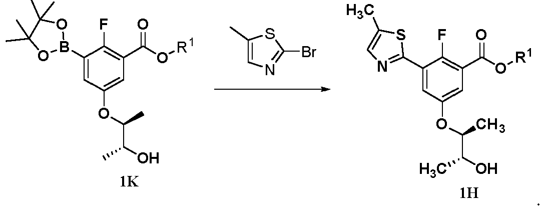

12. The preparation method according to claim 11, wherein
R¹ is C₁-C₄ alkyl or benzyl, such as methyl or ethyl;
and/or the solvent is water, an alcohol solvent, an aromatic hydrocarbon solvent, an ether solvent, or a mixture of any two or more thereof, preferably a mixture of an ether solvent and water, and more preferably a mixture of 1,4-dioxane and water;
and/or the palladium catalyst may be one or more of Pd(OAc)₂, PdCb, Pd(TFA)₂, Pd[O₂C(CH₃)₃]₂, Pd₂(dba)₃, PdBr₂, and Pd(dppf)Cl₂; preferably, the palladium catalyst is Pd(dppf)Cl₂;
and/or the base may be one or more of KHCO₃, NaHCO₃, Na₂CO₃, Ba(OH)₂, K₃PO₄, Cs₂CO₃, K₂CO₃, NaOH, KOH, Et₃N, DIPEA, NaOMe, NaOEt, t-BuOK, t-BuONa, NaH, LiHMDS, NaHMDS, potassium acetate, sodium tert-amylate, n-butyllithium, diethylamine, and dicyclohexylamine; preferably, the base is K₂CO₃;
and/or a molar ratio of intermediate 1K to the palladium catalyst is 1:(0.01-0.1), preferably 1:(0.01-0.05), and more preferably 1:0.05;
and/or a molar ratio of intermediate 1K to 2-bromo-5-methylthiazole is 1:(1-5), preferably 1:(1-2), and more preferably 1:1.1;
and/or the coupling reaction is carried out under an atmosphere of an inert gas, and the inert gas may be nitrogen, helium, or argon;
and/or a reaction temperature for the coupling reaction may be 85 °C to 95 °C, such as 90 °C;
and/or a reaction time for the coupling reaction may be 1 h to 20 h;
and/or after the completion of the coupling reaction, a post-treatment step may be further comprised, for example: subjecting a reaction liquid to cooling, purification by silica gel column, and concentration to give pure compound 1H.

13. The preparation method according to any one of claims 11-12, wherein the preparation method may further comprise a method for preparing compound 1K comprising the following step:
c2) subjecting compound 1J to a coupling reaction as shown below with bis(pinacolato)diboron in the presence of a palladium catalyst and a base in a solvent to give compound 1K:

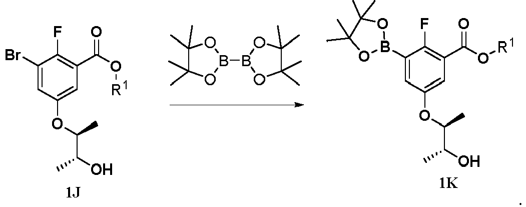

14. The preparation method according to claim 13, wherein
R¹ is C₁-C₄ alkyl or benzyl, such as methyl or ethyl;
and/or the solvent is water, an alcohol solvent, an aromatic hydrocarbon solvent, an ether solvent, or a mixture of any two or more thereof, preferably an ether solvent, and more preferably 1,4-dioxane;
and/or the palladium catalyst may be one or more of Pd(OAc)₂, PdCb, Pd(TFA)₂, Pd[O₂C(CH₃)₃]₂, Pd₂(dba)₃, PdBr₂, and Pd(dppf)Cl₂; preferably, the palladium catalyst is Pd(dppf)Cl₂;
and/or the base may be one or more of KHCO₃, NaHCO₃, Na₂CO₃, Ba(OH)₂, K₃PO₄, Cs₂CO₃, K₂CO₃, NaOH, KOH, Et₃N, DIPEA, NaOMe, NaOEt, t-BuOK, t-BuONa, NaH, LiHMDS, NaHMDS, potassium acetate, sodium tert-amylate, n-butyllithium, diethylamine, and dicyclohexylamine; preferably, the base is potassium acetate;
and/or a molar ratio of intermediate 1J to the palladium catalyst is 1:(0.01-0.1), preferably 1:(0.01-0.05), and more preferably 1:0.05;
and/or a molar ratio of intermediate 1J to bis(pinacolato)diboron is 1:(1-5), preferably 1:(1-1.2), and more preferably 1:1.1;
and/or the coupling reaction is carried out under an atmosphere of an inert gas, and the inert gas may be nitrogen, helium, or argon;
and/or a reaction temperature for the coupling reaction may be 85 °C to 95 °C, such as 90 °C;
and/or a reaction time for the coupling reaction may be 1 h to 20 h;
and/or after the completion of the coupling reaction, a post-treatment step may be further comprised, for example: subjecting a reaction liquid to cooling, purification by silica gel column, and concentration to give pure compound 1K.

15. The preparation method according to any one of claims 13-14, wherein the preparation method may further comprise a method for preparing compound 1J comprising the following step:
c1) subjecting compound 1C to a substitution reaction as shown below with compound 1F in the presence of a base in a solvent, followed by acidification with an acid, to give compound 1J:

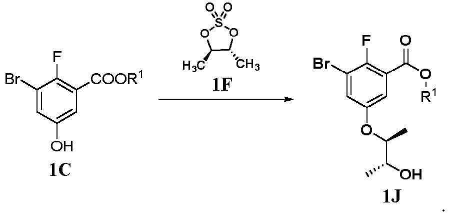

16. The preparation method according to claim 15, wherein
R¹ is C₁-C₄ alkyl or benzyl, such as methyl or ethyl;
and/or the solvent is water, an alcohol solvent, an aromatic hydrocarbon solvent, an ether solvent, or a mixture of any two or more thereof, preferably an ether solvent, and more preferably tetrahydrofuran;
and/or the base may be one or more of DIPEA, triethylamine, pyridine, cesium carbonate, potassium carbonate, potassium phosphate, potassium acetate, sodium hydride, sodium hydroxide, and potassium hydroxide; preferably, the base is potassium carbonate;
and/or the acid may be sulfuric acid, preferably a 20%-75% aqueous sulfuric acid solution, and more preferably a 20% aqueous sulfuric acid solution;
and/or a molar ratio of compound 1C to the acid is 1:(1-5), preferably 1:2;
and/or a molar ratio of compound 1C to compound 1F is 1:(1-5), preferably 1:(1.1-1.3), and more preferably 1:1.2; and/or a molar ratio of compound 1C to the base is 1:(1-5), preferably 1:(1-2), and more preferably 1:1.5;
and/or a reaction temperature for the substitution reaction may be 45 °C to 55 °C, preferably 50 °C;
and/or a temperature for the acidification is 50 °C to 70 °C, preferably 55 °C to 65 °C;
and/or a reaction time for the substitution reaction may be 1 h to 20 h;
and/or after the completion of the substitution reaction, a post-treatment step may be further comprised, for example:
subjecting a reaction liquid to cooling, acidification, extraction, washing, drying, and purification by silica gel column to give pure compound 1H;
preferably, step c1 comprises the following steps: adding compound 1C, compound 1F, and a base to a solvent for a first-stage reaction, and then adding an acid to the reaction system for acidification to give compound 1H.

17. The preparation method according to any one of claims 9, 10, 15, and 16, wherein the preparation method may further comprise a method for preparing compound 1C comprising the following step:
d2) subjecting compound 1B to an esterification reaction as shown below with R¹-OH in the presence of a catalyst in a solvent to give compound 1C:

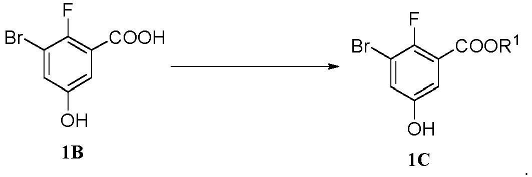

18. The preparation method according to claim 17, wherein
the solvent is water, an alcohol solvent, an aromatic hydrocarbon solvent, an ether solvent, or a mixture of any two or more thereof, preferably an alcohol solvent, and more preferably methanol;
and/or R¹ in R¹-OH is C₁-C₄ alkyl or benzyl, such as methyl or ethyl;
and/or the catalyst may be sulfuric acid, phosphoric acid, thionyl chloride, or acetyl chloride; preferably, the catalyst is acetyl chloride;
and/or the solvent is methanol, the catalyst is acetyl chloride, and R¹-OH is methanol;
and/or a molar ratio of compound 1B to the catalyst is 1:(0.1-1), preferably 1:(0.5-1), and more preferably 1:1; and/or a reaction temperature for the esterification reaction may be 55 °C to 65 °C, such as 60 °C;
and/or after the completion of the esterification reaction, a post-treatment step may be further comprised, for example: subjecting a reaction liquid to cooling, ice water to quench the reaction, centrifugation, and drying to give pure compound 1C.

19. The preparation method according to any one of claims 17-18, wherein the preparation method may further comprise a method for preparing compound 1B comprising the following step:
d1) subjecting compound 1A to a substitution reaction as shown below with water in the presence of a cuprous catalyst and an inorganic base in a solvent to give compound 1B:

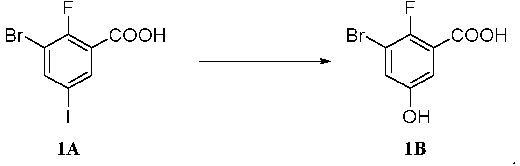

20. The preparation method according to claim 19, wherein
the solvent is water, an alcohol solvent, an aromatic hydrocarbon solvent, an ether solvent, or a mixture of any two or more thereof, preferably water;
and/or the cuprous catalyst may be one or more of copper(I) bromide, copper(I) chloride, copper(I) iodide, and copper(I) oxide; preferably, the cuprous catalyst is copper(I) oxide;
and/or the inorganic base may be one or more of KHCO₃, NaHCO₃, Na₂CO₃, Ba(OH)₂, K₃PO₄, Cs₂CO₃, K₂CO₃, NaOH, KOH, and NaH; preferably, the inorganic base is NaOH;
and/or a molar ratio of intermediate 1A to the cuprous catalyst is 1:(0.1-2), preferably 1:0.2;
and/or a molar ratio of intermediate 1A to the inorganic base is 1:(1-5), preferably 1:(3-5), and more preferably 1:5; and/or a reaction temperature for the substitution reaction may be 90 °C to 100 °C, such as 95 °C;
and/or a reaction time for the substitution reaction may be 1 h to 20 h, such as 10 h;
and/or after the completion of the substitution reaction, a post-treatment step may be further comprised, for example: subjecting a reaction liquid to cooling, filtration through celite, washing, pH adjustment with diluted hydrochloric acid until pH = 1 to 3, extraction, and concentration to give pure compound 1H.
